# EUROPEAN PATENT SPECIFICATION

(11) **EP 1 869 175 B1**
(45) Date of publication and mention of the grant of the patent: **08.08.2012**
(21) Application number: 06796828.9
(22) Date of filing: 21.08.2006
(51) Int. Cl.: C12N 9/88, C12C 11/00, C12G 3/02

(54) **CYSTEINE SYNTHASE GENE AND USE THEREOF**
CYSTEIN SYNTHASE GEN UND SEINE VERWENDUNG
GENE DE LA CYSTEINE SYNTHASE ET APPLICATIONS

(30) Priority: 22.08.2005 JP 2005240350; 23.02.2006 JP 2006047554
(43) Date of publication of application: 26.12.2007
(73) Proprietor: Suntory Holdings Limited, Kita-ku, Osaka-shi, Osaka 530-8203 (JP)
(72) Inventor: NAKAO, Yoshihiro, Mishima-gun, Osaka 6188503 (JP); KODAMA, Yukiko, Mishima-gun, Osaka 6188503 (JP); SHIMONAGA, Tomoko, Mishima-gun, Osaka 6188503 (JP)
(74) Representative: Vossius & Partner
(86) International application number: PCT/JP2006/316785
(87) International publication number: WO 2007/023973

(56) References cited:
- JP-A- 5 244 955
- DATABASE UniProt 1 October 1996 (1996-10-01), "Putative cysteine synthase (EC 2.5.1.47) YGR012W" XP002404863 retrieved from UNIPROT Database accession no. P53206
- DATABASE UniProt 5 July 2004 (2004-07-05), "Cysteine synthase (EC 2.5.1.47) AGR021C" XP002404864 retrieved from UNIPROT Database accession no. Q750D4
- DATABASE UniProt 19 July 2004 (2004-07-19), "Cysteine synthase (EC 2.5.1.47) CAGL0F08789G" XP002404865 retrieved from UNIPROT Database accession no. Q6FTU3
- DATABASE UniProt 16 August 2004 (2004-08-16), "Similar to sp P53206 Saccharomyces cerevisiae YGR012w KLLA0C18029G" XP002404866 retrieved from UNIPROT Database accession no. Q6CST5
- SAITO KAZUKI ET AL: "Isolation and characterization of cDNA that encodes a putative mitochondrion-localizing isoform of cysteine synthase (O-acetylserine(thiol)-lyase) from Spinacia oleracea" JOURNAL OF BIOLOGICAL CHEMISTRY, vol. 269, no. 45, 1994, pages 28187-28192, XP002404797 ISSN: 0021-9258
- ONO BUN-ICHIRO ET AL: "Cysteine biosynthesis in Saccharomyces cerevisiae: A new outlook on pathway and regulation" YEAST, vol. 15, no. 13, 30 September 1999 (1999-09-30), pages 1365-1375, XP002404798 ISSN: 0749-503X

## Description

### TECHNICAL FIELD

The present invention relates to a cysteine synthase gene and to uses of the gene. The invention relates in particular to a brewer's yeast which produces alcoholic beverages of excellent flavor and a method of producing such alcoholic beverages. More specifically, the invention relates to a yeast which improves the flavor of product by increasing the level of expression of the non-ScYGR012W gene characteristic to beer yeast and to a method of producing alcoholic beverages using such a yeast or using a yeast comprising a vector comprising the SCYGR012W gene.

### BACKGROUND ART

The beer yeast used in the production of commercial Pilsner-type light-colored beers has the property of forming hydrogen sulfide during the primary fermentation step. This hydrogen sulfide is one cause of the immature beer aroma that is undesirable for beer quality. To reduce this aroma below a threshold level, extension of secondary fermentation period or extension of maturation period is carried out.

Research on the factors affecting the formation of hydrogen sulfide, (Jangaard, N.O., Gress, H.S. and Coe, R.W.: Amer. Soc. Brew. Chem. Proc., p. 46 (1973); Kuroiwa, Y. and Hashimoto, N.: Brew. Dig., 45, 44 (1970); Hysert, D.W. and Morrison, N.M.: J. Amer. Soc. Brew. Chem., 34, 25 (1976)), and research on the development of a low hydrogen sulfide producing yeast using a mutation process or a cell fusion process (Molzahm, S.W.: J. Amer. Soc. Brew. Chem., 35, 54 (1977)) for lowering the hydrogen sulfide level in beer have been reported.

All of these methods not only reduces the amount of hydrogen sulfide produced by yeast but also affects the other brewing properties of the yeast (fermentation rate, beer flavor). Hence, such a yeast that is well-suited for brewing beer has not been achieved yet. Recently, development of brewer's yeasts using genetic engineering technology has been carried out. Japanese Patent Application Laid-open No. H5-244955 discloses that a beer yeast in which a DNA fragment coding for cystathionine β-synthase has been inserted reduces the production of hydrogen sulfide. However, the degree of reduction was small. That is to say, the amount of hydrogen sulfide produced by the transformant was about 60 to 80% of that produced by the parent strain.

In yeast metabolism, hydrogen sulfide is produced in the process of reducing sulfate ions (SO₄²-) taken up from the medium. This metabolic system is a pathway for the biosynthesis of sulfur-containing amino acids such as methionine and cysteine. Detailed studies have been published on the enzyme engaged in each stage of the pathway and its gene (MET17 gene) (see Tabor, H. and Tabor, C.W, eds., Methods in Enzymology, Vol. 17B (London: Academic Press, 1971); and Jakoby, W.B. and Griffith, O.W., eds., Methods in Enzymology, Vol. 143 (London: Academic Press,1987)).

O-acetylhomoserinesulfhydorelace is an enzyme which transfers a sulfur atom from hydrogen sulfide to O-acetylhomoserine, and is encoded by the MET17 gene. This enzyme also transfers a sulfur atom to O-acetylserine. It has been reported that a beer yeast strain in which the MET17 gene from *Saccharomyces cerevisiae* X2180-1A has been constitutively expressed produces reduced amount of hydrogen sulfide, which is about 2% of the level in the parent strain (Japanese Patent Application Laid-open No.H7-303475).

Further, cysteine synthase is a enzyme which transfers a sulfur atom from a hydrogen sulfide to O-acetyl-L-serine. A gene of the enzyme have not been identified in *Saccharomyces cerevisiae,* although it has been identified in other microorganisms.

Recently, a number of genome sequences were determined. Identification of a gene and estimation of a function of the gene by comparison with known genes were performed based on the determined genome sequences. Further, orthologous genes (i.e., a pair of genes of two different species, wherein the genes are originated from the same gene of a shared ancestry, and current function of the genes are the same.) were estimated by comparison analysis of a numerous genes present in numerous genome sequences of organisms. A gene encoding a cysteine synthase in *Saccharomyces cerevisiae* is estimated to be YGR012W by orthologue analysis of microorganism genome (R. L. Tatusov., M Y. Galperin, D. A. Natale., E.V Koonin; Nucleoc. Acids. Research, 28, 33, 2000). However, a function of the gene has not been confirmed experimentally. Further, it has not been determined if the gene is related to the production amount of hydrogen sulfide.

### DISCLOSURE OF INVENTION

As noted above, variant strains have been developed in order to lower the amount of hydrogen sulfide produced in the final product. As a result, unexpected delays in fermentation and increases in undesirable flavor components was observed in some cases, which makes the practical use of such yeasts questionable. There were thus demands for a method of developing yeasts which produces less hydrogen sulfide without compromising either the fermentation rate or the product quality.

The present inventors made exhaustive studies to solve the above problems, and as a result succeeded in identifying and isolating a gene encoding a cysteine synthase from lager brewing yeast. Moreover, a yeast in which the obtained gene was transformed and expressed was produced to confirm reduction of the amount of hydrogen sulfide production, thereby completing the present invention.

Thus, the present invention relates to a novel cysteine synthase gene existing in a lager brewing yeast, to a protein encoded by said gene, to a transformed yeast in which the expression of said gene is controlled, to a method for controlling the amount of hydrogen sulfide production in a product by using a yeast in which the expression of said gene is controlled. More specifically, the present invention provides the following polynucleotides, a vector comprising said polynucleotide, a transformed yeast introduced with said vector, a method for producing alcoholic beverages by using said transformed yeast, and the like.
(1) A polynucleotide selected from the group consisting of:
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with one to 15 amino, acids thereof being deleted, substituted, inserted and/or added, and having a cysteine synthase activity; and
   (d) a polynucleotide comprising a polynucleotide encoding a protein having an amino acid sequence having 96% or higher identity with the amino acid sequence of SEQ ID NO:2, and having a cysteine synthase activity.
(2) The polynucleotide of (1) above selected from the group consisting of:
   (g) a polynucleotide encoding a protein consisting of the amino acid sequence of SEQ ID NO: 2, or encoding an amino acid sequence of SEQ ID NO: 2 wherein 1 to 5 amino acids thereof is deleted, substituted, inserted, and/or added, and wherein said protein has a cysteine synthase activity;
   (h) a polynucleotide encoding a protein having 97% or higher identity with the amino acid sequence of SEQ ID NO: 2, and having a cysteine synthase activity.
(3) The polynucleotide of (1) above comprising a polynucleotide consisting of SEQ ID NO: 1.
(4) The polynucleotide of (1) above comprising a polynucleotide encoding a protein consisting of SEQ ID NO: 2.
(5) The polynucleotide of any one of (1) to (4) above, wherein the polynucleotide is DNA.
(6) A protein encoded by the polynucleotide of any one of (1) to (5) above.
(7) A vector comprising the polynucleotide of any one of (1) to (5) above.
(8) A yeast, wherein the vector of (7) above is introduced.
(9) A method for producing an alcoholic beverage comprising culturing the yeast of (8) or a yeast comprising the vector comprising the polynucleotide selected from the group consisting of
   (j) a polynucleotide encoding a protein consisting of the amino acid sequence of SEQ ID NO: 6, or encoding an amino acid sequence of SEQ ID NO: 6 wherein 1 to 2 amino acids thereof is deleted, substituted, inserted, and/or added, and wherein said protein has a cysteine synthase activity; and
   (k) a polynucleotide encoding a protein having 99.6% or higher identity with the amino acid sequence of SEQ ID NO: 6, and having a cysteine synthase activity.
(10) The method for producing an alcoholic beverage of (9) above, wherein the brew is a malt beverage.
(11) The method for producing an alcoholic beverage of (9) above, wherein the brew is a wine.

### BRIEF DESCRIPTION OF DRAWINGS

Figure 1 shows the cell growth with time upon beer brewing testing. The horizontal axis represents fermentation time while the vertical axis represents optical density at 660 nm (OD660).
Figure 2 shows the extract consumption with time upon test brew of beer. The horizontal axis represents fermentation time while the vertical axis represents apparent extract concentration (w/w%).
Figure 3 shows the expression behavior of non-ScYGR012W gene in yeasts upon test brew of beer. The horizontal axis represents fermentation time while the vertical axis represents the intensity of detected signal.
Figure 4 shows the cell growth with time upon test brew of beer using parent strain and non-ScYGR012W highly expressed strain. The horizontal axis represents fermentation time while the vertical axis represents optical density at 660 nm (OD660).
Figure 5 shows the sugar consumption with time upon test brew of beer using parent strain and non-ScYGR012W highly expressed strain. The horizontal axis represents fermentation time while the vertical axis represents apparent extract concentration (w/w%).
Figure 6 shows the cell growth with time upon test brew of beer. The horizontal axis represents fermentation time while the vertical axis represents optical density at 660 nm (OD660).
Figure 7 shows the extract consumption with time upon test brew of beer. The horizontal axis represents fermentation time while the vertical axis represents apparent extract concentration (w/w%).
Figure 8 shows the expression behavior of ScYGR012W gene in yeasts upon test brew of beer. The horizontal axis represents fermentation time while the vertical axis represents the intensity of detected signal.
Figure 9 shows the cell growth with time upon test brew of beer using parent strain and ScYGR012W highly expressed strain. The horizontal axis represents fermentation time while the vertical axis represents optical density at 660 nm (OD660).
Figure 10 shows the extract consumption with time upon test brew of beer using parent strain and ScYGR012W highly expressed strain. The horizontal axis represents fermentation time while the vertical axis represents apparent extract concentration (w/w%).

### BEST MODES FOR CARRYING OUT THE INVENTION

The present inventors conceived that it is possible to lower hydrogen sulfide effectively by increasing a cysteine synthase activity of the yeast The present inventors have studied based on this conception and as a result, isolated and identified non-ScYGR012W gene encoding a cysteine synthase unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2. In addition, the present inventors have isolated and identified ScYGR012W gene encoding a cysteine synthase of lager brewing yeast. The nucleotide sequence of the gene is represented by SEQ ID NO: 5. Further, an amino acid sequence of a protein encoded by the gene is represented by SEQ ID NO: 6.

### 1. Polynucleotide of the invention

First of all, the present invention provides (a) a polynucleotide comprising a polynucleotide of the nucleotide sequence of SEQ ID NO:1; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a cysteine synthase gene derived from lager brewing yeast and may include other polynucleotides encoding proteins having equivalent functions to said protein. Proteins with equivalent functions include, for example, (c) a protein of an amino acid sequence of SEQ ID NO: 2 amino acids thereof being deleted, substituted, inserted and/or added and having cysteine synthase activity.

Such proteins include a protein consisting of an amino acid sequence of SEQ ID NO: 2 with, for example, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, 1 to 2, or 1 amino acid residues thereof being deleted, substituted, inserted and/or added and having a cysteine synthase activity. Also disclosed is a protein consisting of an amino acid sequence of SEQ ID NO: 6 with 1 to 2 amino acid residues thereof being deleted, substituted, inserted and/or added and having a cysteine activity. In general, the number of deletions, substitutions, insertions, and/or additions is preferably smaller. In addition, such proteins include (d) a protein having an amino acid sequence with about 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2 and having a cysteine synthase activity. Also enclosed are proteins having an amino acid sequence with 99.6% or higher identity with the amino acid sequence of SEQ ID NO: 6 and having a cysteine synthase activity. In general, the percentage identity is preferably higher.

Cysteine synthase activity may be measured, for example, by a method of Thomas et al. as described in J. Biol. Chem. 45: 28187-28192 (1994).

Furthermore, the present disclosure also contemplates (e) a polynucleotide comprising a polynucleotide which hybridizes to a polynucleotide consisting of a nucleotide sequence complementary to the nucleotide sequence of SEQ ID NO: 1 or SEQ ID NO: 5 under stringent conditions and which encodes a protein having cysteine synthase activity; and (f) a polynucleotide comprising a polynucleotide which hybridizes to a polynucleotide complementary to a nucleotide sequence of encoding a protein of SEQ ID NO: 2 or SEQ ID NO: 6 under stringent conditions, and which encodes a protein having cysteine synthase activity.

Herein, "a polynucleotide that hybridizes under stringent conditions" refers to a polynucleotide, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or SEQ ID NO: 5, or polynucleotide encoding the amino acid sequence of SEQ ID NO: 2 or SEQ ID NO: 6 as a probe. The hybridization method may be a method described, for example, in MOLECULAR CLONING 3rd Ed., CURRENT PROTOCOLS IN MOLECULAR BIOLOGY, John Wiley & Sons 1987-1997.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91 % or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotides encoding the amino acid sequence of SEQ ID NO: 2 or SEQ ID NO: 6 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a) to (1) above. A preferred protein of the present invention comprises an amino acid sequence of SEQ ID NO:2 with one to 15 amino acids thereof being deleted, substituted, inserted and/or added, and has a cysteine synthase activity. Also disclosed is a protein comprising an amino acid sequence of SEQ ID NO: 6 with 1 to 2 amino acids thereof being deleted, substituted, inserted and/or added and having a cysteine synthase activity.
Such protein includes those having an amino acid sequence of SEQ ID NO: 2 or SEQ ID NO: 6 with amino acid residues thereof of the number mentioned above being deleted, substituted, inserted and/or added and having a cysteine synthase activity. In addition, such protein includes those having homology as described above with the amino acid sequence of SEQ ID NO: 2 or SEQ ID NO: 6 and having a cysteine synthase activity.
Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides (for example, DNA) described in (a) to (1) above. Generally, the vector of the present invention comprises an expression cassette including as components (x) a promoter that can transcribe in a yeast cell; (y) a polynucleotide (for example, DNA) described in any of (a) to (1) above that is linked to the promoter in sense or antisense direction; and (z) a signal that functions in the yeast with respect to transcription termination and polyadenylation of DNA molecule. According to the present invention, in order to highly express the protein of the invention described above upon brewing alcoholic beverages (e.g., beer) described below, these polynucleotides are introduced into the promoter in the sense direction to promote expression of the polynucleotide (for example, DNA) described in any of (a) to (i) above.

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3 phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a celulenin-resistant gene (fas2m, PDR4) (Junji Inokosbi et al., Biochemistry, 64, 660, 1992; and Hussain et aL, Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/10, *Saccharomyces carlsbergensis* NCYC453 or NCYC456, or *Saccharomyces cerevisiae* NBRC1951, NBRC1952, NBRC1953 or NBRC1954 may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad Sci. USA, 75: 1929(1978)), a lithium acetate method (J. Bacteriology, 153: 163 (1983)), and methods described in Proc. Natl. Acad Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce a desired alcoholic beverage with enhanced flavor with a lowered content of hydrogen sulfide. In addition, yeasts to be selected by the yeast assessment method of the present invention described below can also be used. The target alcoholic beverages include, for example, but not limited to beer, sparkling liquor (*happoushu*) such as a beer-taste beverage, wine, whisky, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with a lowered content of hydrogen sulfide. Thus, according to the present invention, alcoholic beverages with enhanced flavor can be produced using the existing facility without increasing the cost

### 5. Yeast assessment method

The present disclosure relates to a method for assessing a test yeast for its hydrogen sulfide-producing capability by using a primer or a probe designed based on a nucleotide sequence of a cysteine synthase gene having the nucleotide sequence of SEQ ID NO:1 or SEQ ID NO: 5. General techniques for such assessment method is known and is described in, for example, WO01/0405 Japanese Laid-Open Patent Application No. 8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the cysteine synthase gene, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product This method allows prediction and assessment of the capability of the yeast to produce hydrogen sulfide as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present disclosure, a test yeast is cultured to measure an expression level of the cysteine synthase gene having the nucleotide sequence of SEQ ID NO: 1 or SEQ ID NO: 5 to assess the test yeast for its hydrogen sulfide-producing capability. In measuring an expression level of the cysteine synthase gene, the test yeast is cultured and then mRNA or a protein resulting from the cysteine synthase gene is quantified. The quantification of mRNA or protein may be carried out by employing a known technique. Messenger RNA (mRNA) may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the cysteine synthase gene having the nucleotide sequence of SEQ ID NO: 1 or SEQ ID NO: 5 are measured to select a test yeast with the gene expression level according to the target capability of producing hydrogen sulfide, thereby selecting a yeast favorable for brewing desired alcoholic beverages. In addition, a reference yeast and a test yeast may be cultured so as to measure and compare the expression level of the gene in each of the yeasts, thereby selecting a favorable test yeast More specifically, for example, a reference yeast and one or more test yeasts are cultured and an expression level of the cysteine synthase gene having the nucleotide sequence of SEQ ID NO: 1 or SEQ ID NO: 5 is measured in each yeast By selecting a test yeast with the gene expressed higher than that in the reference yeast, a yeast suitable for brewing desired alcoholic beverages can be selected.

Alternatively, test yeasts are cultured and a yeast with a lower hydrogen sulfide-producing capability or with a higher or lower cysteine synthase activity is selected, thereby selecting a yeast suitable for brewing desired alcoholic beverages.

In these cases, the test yeasts or the reference yeast may be, for example, a yeast introduced with the vector of the invention, a yeast in which an expression of a polynucleotide (DNA) of the invention has been controlled, an artificially mutated yeast or a naturally mutated yeast. The production amount of hydrogen sulfide can be measured by, for example, any of the methods described in Brauwissenschaft. 31. 1 (1978), Applied. Environm. Microbiol. 66: 4421-4426 (2000), or J. Am. Soc. Brew. Chem. 53: 58-62 (1995). cysteine synthase activity can be measured by, for example, a method described in J. Biol. Chem. 45: 28187-28192 (1994). The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus, S. cerevisiae,* and *S. carlsbergensis*). According to the present disclosure, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of a novel cysteine synthase (non-ScYGR012W) Gene

A specific novel cysteine synthase gene non-ScYGR012W (SEQ ID NO: 1) of a lager brewing yeast were found as a result of a search utilizing the comparison database described in Japanese Patent Application Laid-Open No. 2004-283169. Based on the acquired nucleotide sequence information, primers non-ScYGR012W for (SEQ ID NO: 3) and non-ScYGR012W_rv (SEQ ID NO: 4) were designed to amplify the full-length genes, respectively PCR was carried out using chromosomal DNA of a genome sequencing strain, *Saccharomyces pastorianus* Weihenstephan 34/70 strain, also abbreviated to "W34/70 strain", as a template to obtain DNA fragments (about 1.2 kb) including the full-length gene of non-ScYGR012W.

The thus-obtained non-ScYGR012W gene fragment was inserted into pCR2.1-TOPO vector (Invitrogen) by TA cloning. The nucleotide sequences of non ScYGR012W gene were analyzed according to Sanger's method (F. Sanger, Science, 214: 1215, 1981) to confirm the nucleotide sequence.

### Example 2: Analysis of Expression of non-ScYGR012W Gene during Beer Fermentation

A fermentation test was conducted using a lager brewing yeast, *Saccharomyces pastorianus* W34/70 strain and then mRNA extracted from yeast cells during fermentation was detected by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

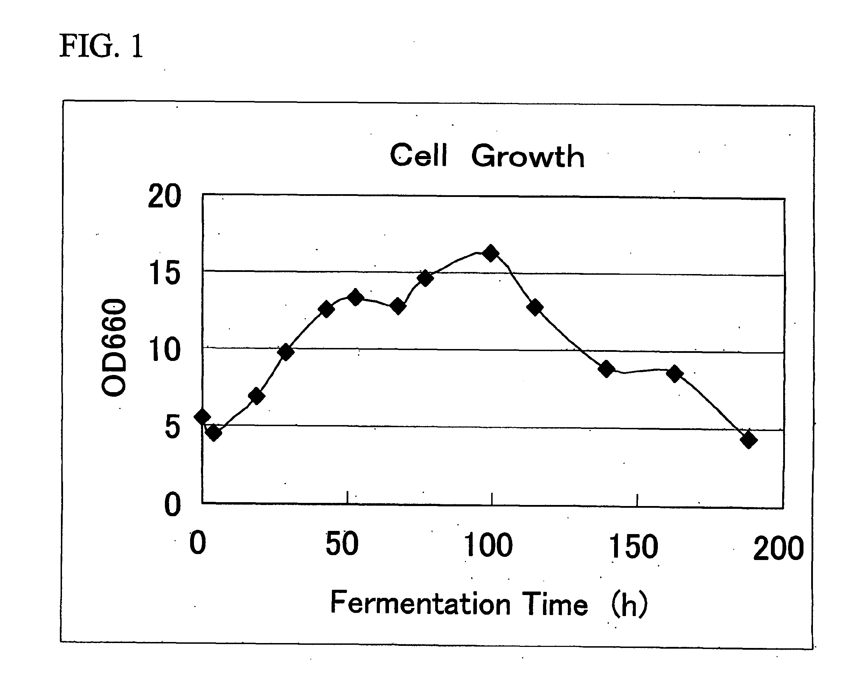

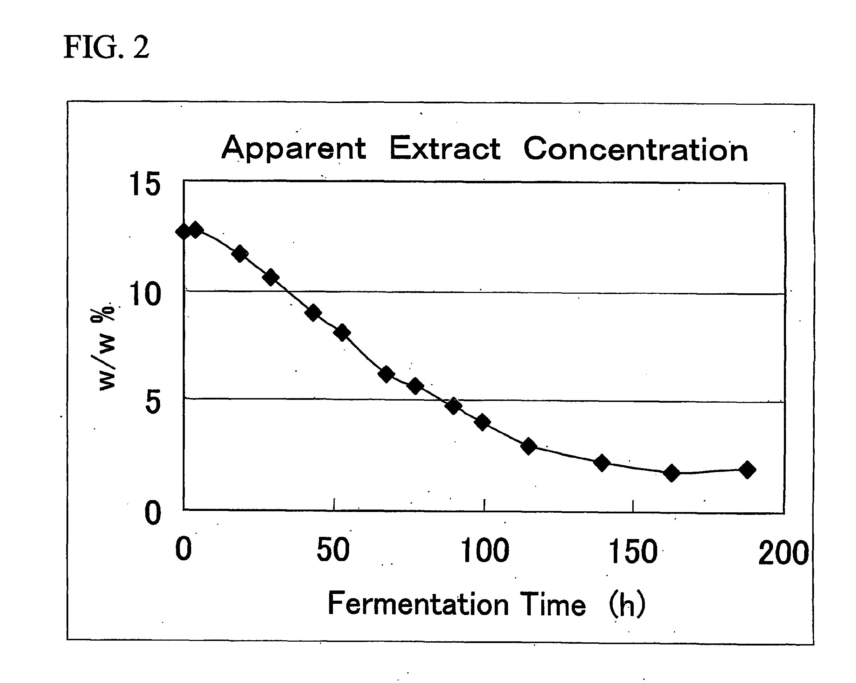

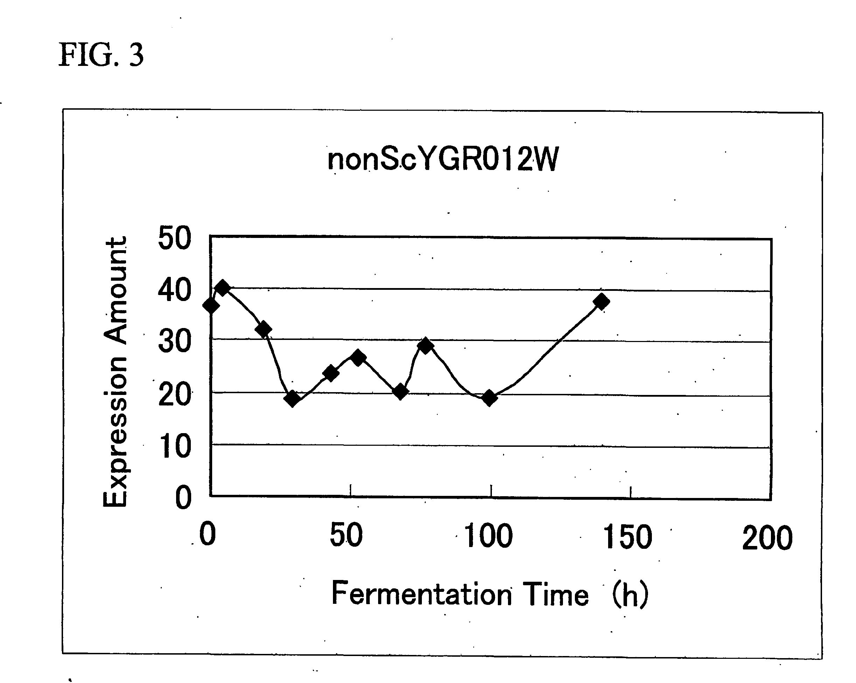

Sampling of fermented liquid was performed with time, and variation with time of yeast growth amount (Fig. 1) and apparent extract concentration (Fig. 2) was observed. Simultaneously, yeast cells were sampled to prepare mRNA, and the prepared mRNA was labeled with biotin and was hybridized to a beer yeast DNA microarray The signal was detected using GCOS; GeneChip Operating Software 1.0 (manufactured by Affymetrix Co.). Expression pattern of non-ScYGR012W gene is shown in Figure 3. As a result, it was confirmed that non ScYGR012W gene was expressed in the general beer fermentation.

### Example 3: High Expression of non-ScYGR012W Gene

The non-ScYGR012W/pCR2.1-TOPO described in Example 1 was digested using the restriction enzymes SacI and NotI so as to prepare a DNA fragment containing the entire length of the protein-encoding region This fragment was ligated to pYCGPYNot treated with the restriction enzymes SacI and NotI, thereby constructing the non-ScYGR012W high expression vector non-ScYGR012W/pYCGPYNot. pYCGPYNot is a YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 4: Analysis of Amount of Hydrogen Sulfide Produced in Test Brew of Beer

A fermentation test was carried out under the following conditions using the parent strain (W34/70 strain) and the non-ScYGR012W highly expressed strain obtained in Example 3.

| | |
|---|---|
| Wort extract concentration | 12% |
| Wort content | 1L |
| Wort dissolved oxygen concentration | approx. 8 ppm |
| Fermentation temperature | 15°C (fixed) |
| Yeast pitching rate | 5 g wet yeast cells/L of wort |

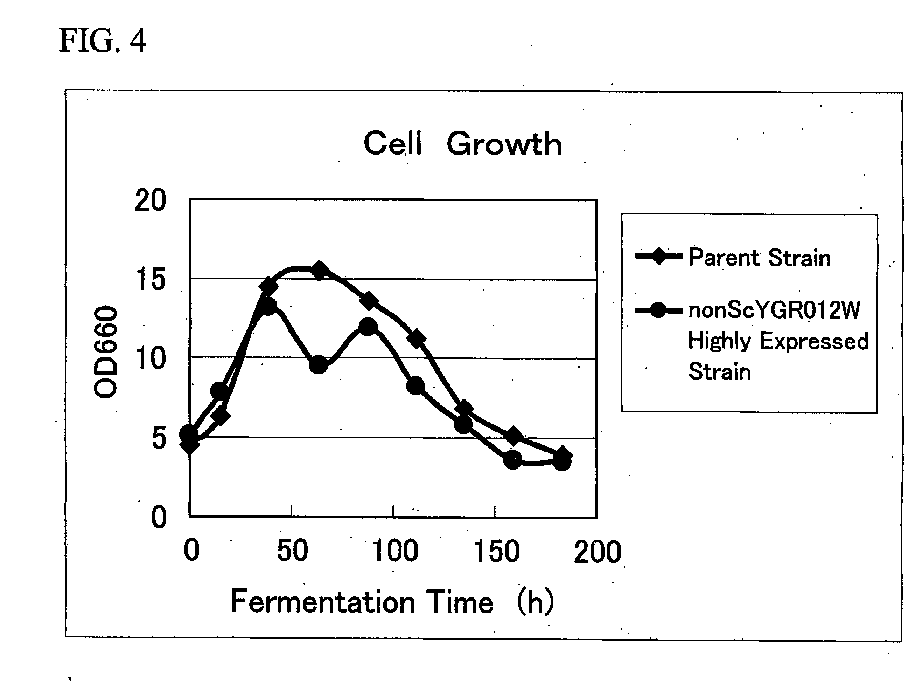

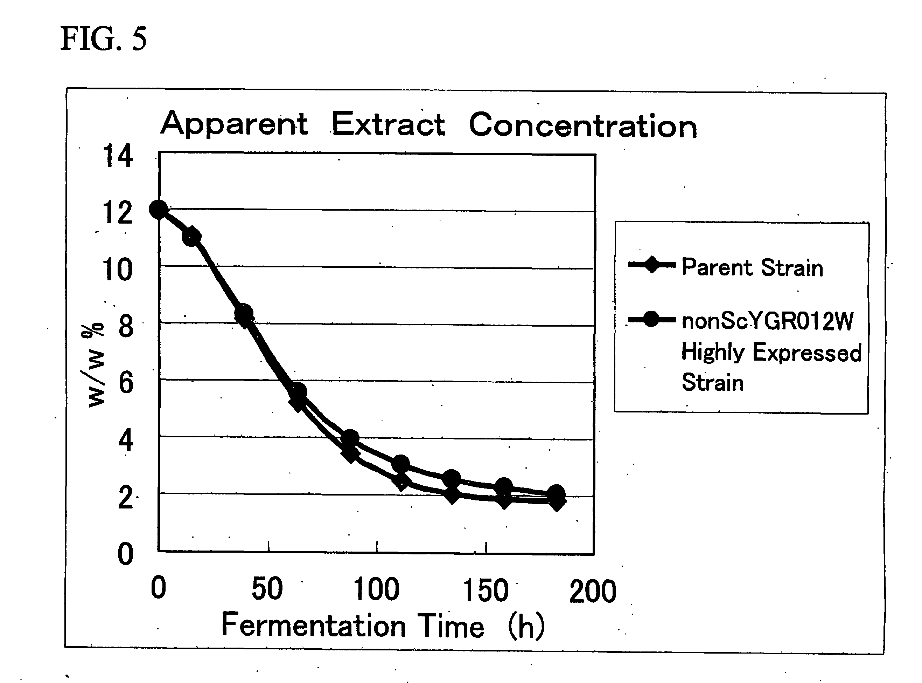

The fermentation broth was sampled over time, and the change over time in the yeast growth rate (OD660) (see FIG. 4) and the amount of extract consumed were determined (see FIG. 5). Quantitative determination of the hydrogen sulfide on completion of fermentation was carried out based on the method of Takahashi et aL (Brauwissenschaft 31, 1 (1978)). First, a sample containing a known concentration of hydrogen sulfide was measured and a standard curve for hydrogen sulfide was prepared from the peak area for the hydrogen sulfide detected. The amount of hydrogen sulfide was determined from the relationship between the standard curve and the area for the hydrogen sulfide detected in measurement of the fermentation broth under the same conditions as those used for analyzing the standard sample (Table 1).

**Table 1. Amount of hydrogen sulfide in fermentation broth at completion of fermentation.**

| | Parent strain (W34/70 strain) | non-ScYGR012W highly expressed strain |
|---|---|---|
| H₂S (ppb) | 22.1 | 3.3 |

The amount of hydrogen sulfide that had been produced on completion of fermentation was 22.1 ppb for the parent strain, whereas it was 3.3 ppb for the non-ScYGR012W highly expressed strain as described in Table 1. It was clear from these results that the amount of hydrogen sulfide production was reduced about 85%by high expression of the non-ScYGR012W gene.

### Example 5: Cloning of a cysteine synthase (ScYGR012W) Gene

A cysteine synthase gene ScYGR012W (SEQ ID NO: 5) of a lager brewing yeast were found as a result of a search utilizing the comparison database described in Japanese Patent Application Laid-Open No. 2004-283169. Based on the acquired nucleotide sequence information, primers ScYGR012W_for (SEQ ID NO: 7) and ScYGR012W_rv (SEQ ID NO: 8) were designed to amplify the full-length genes, respectively. PCR was carried out using chromosomal DNA of a genome sequencing strain, *Saccharomyces pastorianus* Weihenstephan 34/70 strain, as a template to obtain DNA fragments (about 1.2 kb) including the full-length gene of ScYGR012W.

The thus-obtained ScYGR012W gene fragment was inserted into pCR2.1-TOPO vector (Invitrogen) by TA cloning. The nucleotide sequences of ScYGR012W gene were analyzed according to Sanger's method (F. Sanger, Science, 214: 1215, 1981) to confirm the nucleotide sequence.

### Example 6: Analysis of Expression of ScYGR012W Gene during Beer Fermentation

A fermentation test was conducted using a lager brewing yeast, *Saccharomyces pastorianus* W34/70 strain and then mRNA extracted from yeast cells during fermentation was analyzed by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8 × 10⁶ cells/mL |

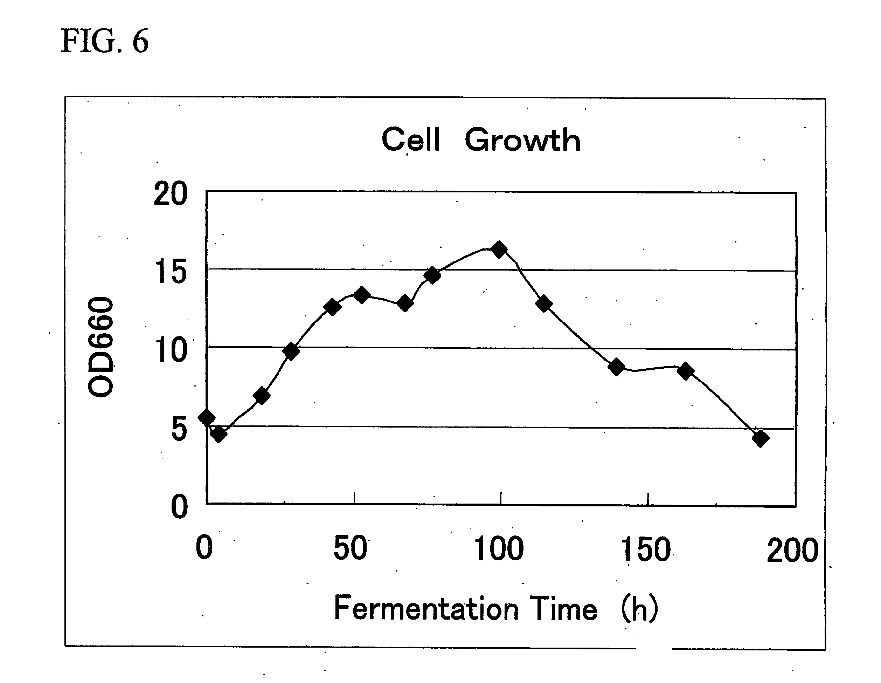

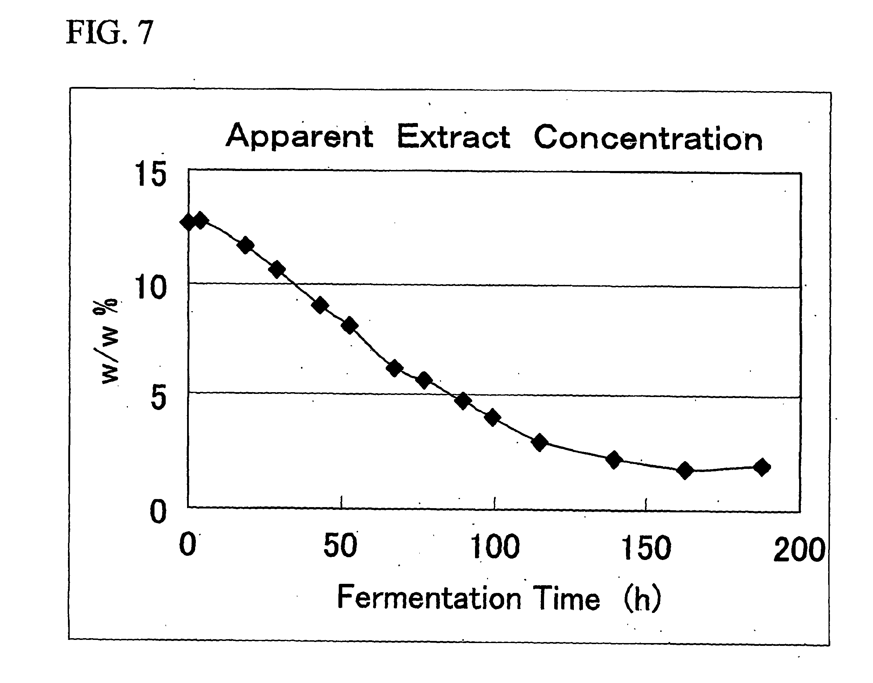

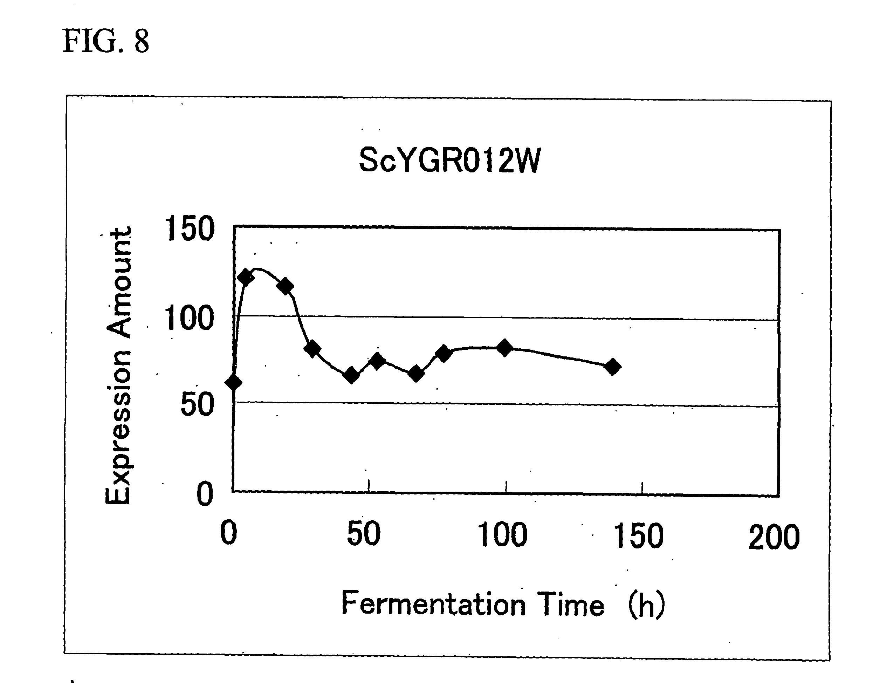

Sampling of fermented liquid was performed with time, and variation with time of yeast growth amount (Fig. 6) and apparent extract concentration (Fig. 7) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of ScYGR012W gene is shown in Figure 8. As a result, it was confirmed that ScYGR012W gene was expressed in the general beer fermentation.

### Example 7: High Expression of ScYGR012W Gene

The ScYGR012W/pCR2.1-TOPO described in Example 5 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the ScYGR012W high expression vector ScYGR012W/pYCGPYNot. pYCGPYNot is the YCp-type yeast expression vector. The inserted gene is highly expressed by the pyruvate kinase gene PYX1 promotor. The geneticin-resistant gene G418^{r} is included as the selection marker in the yeast, and the ampicillin-resistant gene Amp^{r} is included as the selection marker in *Escherichia coli*.

Using the high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 8: Analysis of Amount of Hydrogen Sulfide Produced in Test Brew of Beer

A fermentation test was carried out under the following conditions using the parent strain (W34/70 strain) and the ScYGR012W high expression strains obtained in Example 7.

| | |
|---|---|
| Wort extract concentration | 12% |
| Wort content | 1 L |
| Wort dissolved oxygen concentration | approx. 8 ppm |
| Fermentation temperature | 15°C (fixed) |
| Yeast pitching rate | 5 g wet yeast cells/L of wort |

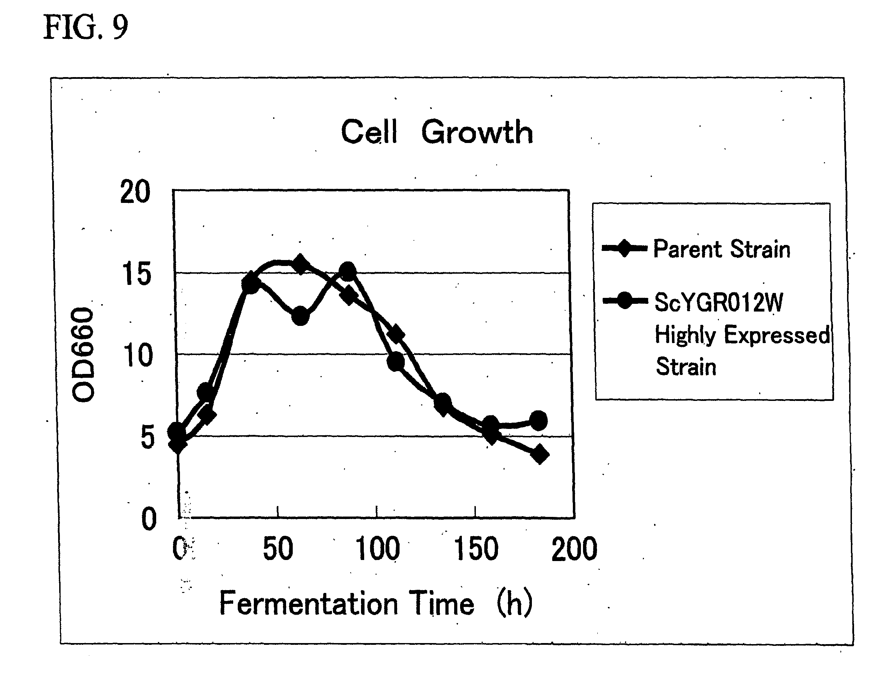

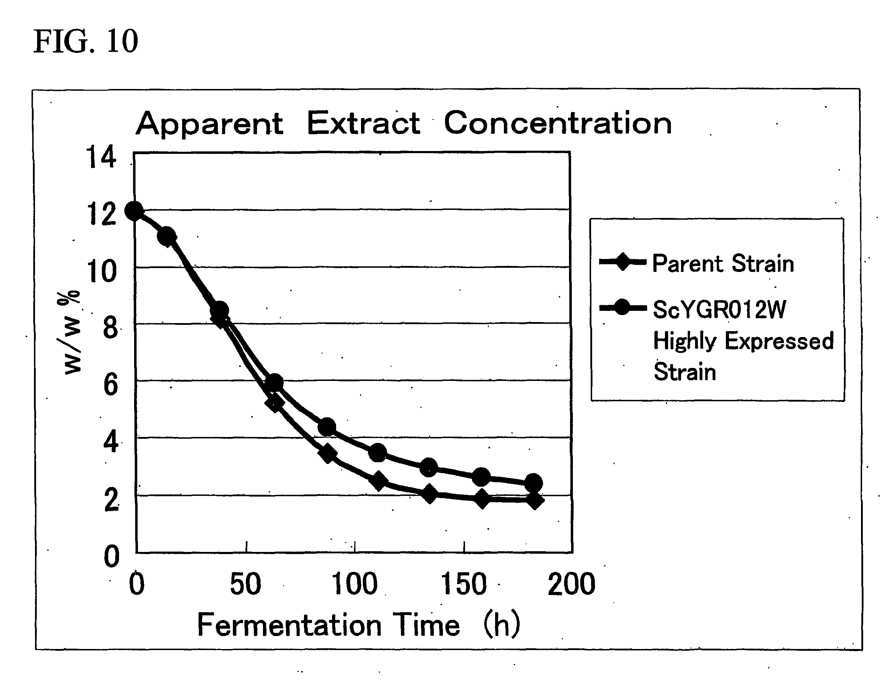

The fermentation broth was sampled over time, and the change over time in the yeast growth rate (OD660) (FIG. 9) and the amount of extract consumed were determined (FIG. 10). Quantitative determination of the hydrogen sulfide on completion of fermentation was carried out based on the method of Takahashi et al. (Brauwissenschaft 31, 1 (1978)). First, a sample containing a known concentration of hydrogen sulfide was measured and a standard curve for hydrogen sulfide was prepared from the peak area for the hydrogen sulfide detected. The amount of hydrogen sulfide was determined from the relationship between the standard curve and the area for the hydrogen sulfide detected in measurement of the fermentation broth under the same conditions as those used for analyzing the standard sample (Table 2).

**Table 2. Amount of hydrogen sulfide in fermentation broth at completion of fermentation.**

| | Parent strain (W34/70 strain) | ScYGR012W highly expressed strain |
|---|---|---|
| H₂S(ppb) | 22.1 | 2.6 |

The amount of hydrogen sulfide that had been produced on completion of fermentation was 22.1 ppb for the parent strain, whereas whereas it was 2.6 ppb for the ScYGR012W highly expressed strains as described in Table 2. It was clear from these results that the amount of hydrogen sulfide production was reduced about 88%by high expression of the ScYGR012W gene.

### INDUSTRIAL APPLICABILITY

The inventive method of producing alcoholic beverages, by holding to a low level the concentration of hydrogen sulfide in beer fermentation and the finished product, can be used to produce alcoholic beverages having an excellent flavor. According to the method for producing alcoholic beverages by using a yeast transformed with a cysteine synthase (The method for producing alcoholic beverages of the present invention), hydrogen sulfide is consumed quickly by the cysteine synthase. Accordingly, concentration of hydrogen sulfide can be lowered in beer fermentation and finished product so that alcoholic beverages with superior flavor can be produced.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 15 amino acids thereof being deleted, substituted, inserted and/or added, and having a cysteine synthase activity; and
(d) a polynucleotide comprising a polynucleotide encoding a protein having an amino acid sequence having 96% or higher identity with the amino acid sequence of SEQ ID NO:2, and having a cysteine synthase activity.

2. The polynucleotide of claim 1 selected from the group consisting of:
(g) a polynucleotide encoding a protein consisting of the amino acid sequence of SEQ ID NO: 2, or encoding an amino acid sequence of SEQ ID NO: 2 wherein 1 to 5 amino acids thereof is deleted, substituted, inserted, and/or added, and wherein said protein has cysteine synthase activity; and
(h) a polynucleotide encoding a protein having 97% or higher identity with the amino acid sequence of SEQ ID NO: 2, and having cysteine synthase activity.

3. The polynucleotide of claim 1 comprising a polynucleotide consisting of SEQ ID NO: 1.

4. The polynucleotide of claim 1 comprising a polynucleotide encoding a protein consisting of SEQ ID NO: 2.

5. The polynucleotide of any one of claims 1 to 4, wherein the polynucleotide is DNA.

6. A protein encoded by the polynucleotide of any one of claims 1 to 5.

7. A vector comprising the polynucleotide of any one of claims 1 to 5.

8. A yeast comprising the vector of claim 7.

9. A method for producing an alcoholic beverage comprising culturing the yeast of claim 8 or a yeast comprising the vector comprising the polynucleotide selected from the group consisting of:
(j) a polynucleotide encoding a protein consisting of the amino acid sequence of SEQ ID NO: 6, or encoding an amino acid sequence of SEQ ID NO: 6 wherein 1 to 2 amino acids thereof is deleted, substituted, inserted, and/or added, and wherein said protein has a cysteine synthase activity; and
(k) a polynucleotide encoding a protein having 99.6% or higher identity with the amino acid sequence of SEQ ID NO: 6, and having a cysteine synthase activity.

10. The method for producing an alcoholic beverage of claim 9, wherein the brewed alcoholic beverage is a malt beverage.

11. The method for producing an alcoholic beverage of claim 9, wherein the brewed alcoholic beverage is wine.

## Patentansprüche

1. Polynucleotid ausgewählt aus der Gruppe bestehend aus:
(a) einem Polynucleotid umfassend ein Polynucleotid, das aus der Nucleotidsequenz von SEQ ID NO:1 besteht;
(b) einem Polynucleotid umfassend ein Polynucleotid, das ein Protein codiert, das aus der Aminosäuresequenz von SEQ ID NO:2 besteht;
(c) einem Polynucleotid umfassend ein Polynucleotid, das ein Protein codiert, das aus der Aminosäuresequenz von SEQ ID NO:2 besteht, in der eine bis 15 Aminosäuren davon entfernt, ersetzt, eingefügt, und/oder hinzugefügt sind, und das eine Cysteinsynthase-Aktivität hat; und
(d) einem Polynucleotid umfassend ein Polynucleotid, das ein Protein codiert, das eine Aminosäuresequenz hat, die eine 96%ige oder höhere Identität mit der Aminosäuresequenz von SEQ ID NO:2 hat, und das eine Cysteinsynthase-Aktivität hat.

2. Polynucleotid nach Anspruch 1 ausgewählt aus der Gruppe bestehend aus:
(g) einem Polynucleotid, das ein Protein codiert, das aus der Aminosäuresequenz von SEQ ID NO:2 besteht, oder eine Aminosäuresequenz nach SEQ ID NO:2 codiert, in der eine bis fünf Aminosäuren davon entfernt, ersetzt, eingefügt, und/oder hinzugefügt sind, und wobei das Protein Cysteinsynthase-Aktivität hat; und
(h) einem Polynucleotid, das ein Protein codiert, das eine 97%ige oder höhere Identität mit der Aminosäuresequenz von SEQ ID NO:2 hat, und das Cysteinsynthase-Aktivität hat.

3. Polynucleotid nach Anspruch 1 umfassend ein Polynucleotid, das aus SEQ ID NO:1 besteht.

4. Polynucleotid nach Anspruch 1 umfassend ein Polynucleotid, das ein Protein codiert, das aus SEQ ID NO:2 besteht.

5. Polynucleotid nach einem der Ansprüche 1 bis 4, wobei das Polynucleotid DNA ist.

6. Protein, das durch das Polynucleotid nach einem der Ansprüche 1 bis 5 codiert wird.

7. Vektor, der das Polynucleotid nach einem der Ansprüche 1 bis 5 umfasst.

8. Hefe, umfassend den Vektor nach Anspruch 7.

9. Verfahren zur Herstellung eines alkoholischen Getränks umfassend die Züchtung der Hefe nach Anspruch 8 oder einer Hefe, umfassend den Vektor, der das Polynucleotid umfasst, das ausgewählt ist aus der Gruppe bestehend aus:
(j) einem Polynucleotid, das ein Protein codiert, das aus der Aminosäuresequenz von SEQ ID NO:6 besteht, oder das eine Aminosäuresequenz von SEQ ID NO:6 codiert, in der eine bis zwei Aminosäuren davon entfernt, ersetzt, eingefügt, und/oder hinzugefügt sind, und wobei das Protein eine Cysteinsynthase-Aktivität hat; und
(k) einem Polynucleotid, das ein Protein codiert, das eine 99,6%-ige oder höhere Identität mit der Aminosäuresequenz von SEQ ID NO:6 hat, und das Cysteinsynthase-Aktivität hat.

10. Verfahren zur Herstellung eines alkoholischen Getränks nach Anspruch 9, wobei das gebraute alkoholische Getränk ein Malzgetränk ist.

11. Verfahren zur Herstellung eines alkoholischen Getränks nach Anspruch 9, wobei das gebraute alkoholische Getränk Wein ist.

## Revendications

1. Polynucléotide choisi dans le groupe consistant en:
(a) un polynucléotide comprenant un polynucléotide consistant en la séquence nucléotidique de SEQ ID NO: 1;
(b) un polynucléotide comprenant un polynucléotide codant une protéine consistant en la séquence d'aminoacides de SEQ I NO:2;
(c) un polynucléotide comprenant un polynucléotide codant une protéine consistant en la séquence d'aminoacides de SEQ ID NO:2, 1 à 15 aminoacides de celle-ci étant délétés, substitués, insérés et/ou ajoutés, et ayant une activité cystéine synthase; et
(d) un polynucléotide comprenant un polynucléotide codant une protéine ayant une séquence d'aminoacides ayant 96 % d'identité ou plus avec la séquence d'aminoacides de SEQ ID NO:2, et ayant une activité cystéine synthase.

2. Polynucléotide selon la revendication 1 choisi dans le groupe consistant en:
(g) un polynucléotide codant une protéine consistant en la séquence d'aminoacides de SEQ ID NO:2, ou codant une séquence d'aminoacides de SEQ ID NO:2 où 1 à 5 aminoacides de celle-ci sont délétés, substitués, insérés et/ou ajoutés, et où ladite protéine a une activité cystéine synthase; et
(h) un polynucléotide codant une protéine ayant 97 % d'identité ou plus avec la séquence d'aminoacides de SEQ ID NO:2, et ayant une activité cystéine synthase.

3. Polynucléotide selon la revendication 1 comprenant un polynucléotide consistant en SEQ ID NO:1.

4. Polynucléotide selon la revendication 1 comprenant un polynucléotide codant une protéine consistant en SEQ ID NO:2.

5. Polynucléotide selon l'une quelconque des revendications 1 à 4, où le polynucléotide est un ADN.

6. Protéine codée par le polynucléotide selon l'une quelconque des revendications 1 à 5.

7. Vecteur comprenant le polynucléotide selon l'une quelconque des revendications 1 à 5.

8. Levure comprenant le vecteur selon la revendication 7.

9. Procédé pour produire une boisson alcoolique comprenant la culture de la levure selon la revendication 8 ou d'une levure comprenant le vecteur comprenant le polynucléotide choisi dans le groupe consistant en:
(j) un polynucléotide codant une protéine consistant en la séquence d'aminoacides de SEQ ID NO:6, ou codant une séquence d'aminoacides de SEQ ID NO:6 où 1 à 2 aminoacides de celle-ci sont délétés, substitués, insérés et/ou ajoutés, et où ladite protéine a une activité cystéine synthase; et
(k) un polynucléotide codant une protéine ayant 99,6 % d'identité ou plus avec la séquence d'aminoacides de SEQ ID NO:6, et ayant une activité cystéine synthase.

10. Procédé pour produire une boisson alcoolique selon la revendication 9, où la boisson alcoolique brassée et une boisson de malt.

11. Procédé pour produire une boisson alcoolique selon la revendication 9, où la boisson alcoolique brassée est le vin.


### SEQUENCE LISTING

<110> Suntory Limited
<120> Cysteine synthase gene and use thereof
<130> PCT06-0075
<150> JP2005-240350
   <151> 2005-08-22
<150> JP2006-47554
   <151> 2006-02-23
<160> 8
<210> 1
   <211> 1188
   <212> DNA
   <213> Yeast
<400> 1
   atgtccagtt cacgagataa gaacgctttg tatttgcctt ggaacgaatg catttccatc 60
   gcttccgtcc taatcggcgc atatgcatcg tataaatatt ataaactatg caaggcgcag 120
   gatataccac gtccaaaaga tggtgtggag cagctaatag gaaacactcc cctggttcag 180
   attagatccc tttccaaggc caccggagtt aacatttatg caaaattaga gttatgtaac 240
   ccagcaggaa gtgctaaaga tagagtagca ttaaacataa taaagaccgc agaggaacga 300
   ggtgagctaa tcagaggcga acctggttgg gtgttcgaag gaacaagcgg ttcaacaggc 360
   atttctatag ccatggtctg taatgcacta ggatataggg cacacatttc tttacccgac 420
   gacacatcgt tagaaaagtt ggcattacta gaaagtctcg gtgctacagt caataaggtt 480
   aagcctgcca gtattgtcga tccaaatcaa tatgttaatg cagctaagaa agcatgcgat 540
   gatttgagta aggaaggtaa tggagtaaag gcagtttttg ctgaccaatt tgaaaatgaa 600
   gccaattgga gagtacacta tcaaaccacg ggtccagaaa ttgcccatca aatggaagga 660
   aaaattgatg cgtttattgc tggctgtggt accggtggaa cgataactgg agtggccaaa 720
   tatctaaagg aaagcgcaaa aattccgtgc catgtcgttc ttgcagatcc acaaggttca 780
   ggcttttata ataggataaa ctacggcgtg atgtatgact atgtagaaaa agaaggtaca 840
   agaaggaggc atcaggtgga tactattgta gaaggtatcg ggctaaacag gattactcaa 900
   aatttccaca tgggcgaaga gtttatcgat gaatcgatac gtgtcaatga cactcaagct 960
   atcagaatgg caaaatacct atcagtaaac gatgggttat tcgtaggaag tagtacagct 1020
   ataaacgcag tagctgctgt tcaaatcgcg aaaagactcc ctcctggtgc caacattgta 1080
   attattgcat gcgacagcgg atcaagacat ttaagtaaat tctggaaaga agctaaacaa 1140
   atagagtatg acatatcgtt aaaggagata acaggtggca ttcgatga 1188
<210> 2
   <211> 395
   <212> PRT
   <213> Yeast
<400> 2
   Met Ser Ser Ser Arg Asp Lys Asn Ala Leu Tyr Leu Pro Trp Asn Glu
   1 5 10 15
   Cys Ile Ser Ile Ala Ser Val Leu Ile Gly Ala Tyr Ala Ser Tyr Lys
   20 25 30
   Tyr Tyr Lys Leu Cys Lys Ala Gln Asp Ile Pro Arg Pro Lys Asp Gly
   35 40 45
   Val Glu Gln Leu Ile Gly Asn Thr Pro Leu Val Gln Ile Arg Ser Leu
   50 55 60
   Ser Lys Ala Thr Gly Val Asn Ile Tyr Ala Lys Leu Glu Leu Cys Asn
   65 70 75 80
   Pro Ala Gly Ser Ala Lys Asp Arg Val Ala Leu Asn Ile Ile Lys Thr
   85 90 95
   Ala Glu Glu Arg Gly Glu Leu Ile Arg Gly Glu Pro Gly Trp Val Phe
   100 105 110
   Glu Gly Thr Ser Gly Ser Thr Gly Ile Ser Ile Ala Met Val Cys Asn
   115 120 125
   Ala Leu Gly Tyr Arg Ala His Ile Ser Leu Pro Asp Asp Thr Ser Leu
   130 135 140
   Glu Lys Leu Ala Leu Leu Glu Ser Leu Gly Ala Thr Val Asn Lys Val
   145 150 155 160
   Lys Pro Ala Ser Ile Val Asp Pro Asn Gln Tyr Val Asn Ala Ala Lys
   165 170 175
   Lys Ala Cys Asp Asp Leu Ser Lys Glu Gly Asn Gly Val Lys Ala Val
   180 185 190
   Phe Ala Asp Gln Phe Glu Asn Glu Ala Asn Trp Arg Val His Tyr Gln
   195 200 205
   Thr Thr Gly Pro Glu Ile Ala His Gln Met Glu Gly Lys Ile Asp Ala
   210 215 220
   Phe Ile Ala Gly Cys Gly Thr Gly Gly Thr Ile Thr Gly Val Ala Lys
   225 230 235 240
   Tyr Leu Lys Glu Ser Ala Lys Ile Pro Cys His Val Val Leu Ala Asp
   245 250 255
   Pro Gln Gly Ser Gly Phe Tyr Asn Arg Ile Asn Tyr Gly Val Met Tyr
   260 265 270
   Asp Tyr Val Glu Lys Glu Gly Thr Arg Arg Arg His Gln Val Asp Thr
   275 280 285
   Ile Val Glu Gly Ile Gly Leu Asn Arg Ile Thr Gln Asn Phe His Met
   290 295 300
   Gly Glu Glu Phe Ile Asp Glu Ser Ile Arg Val Asn Asp Thr Gln Ala
   305 310 315 320
   Ile Arg Met Ala Lys Tyr Leu Ser Val Asn Asp Gly Leu Phe Val Gly
   325 330 335
   Ser Ser Thr Ala Ile Asn Ala Val Ala Ala Val Gln Ile Ala Lys Arg
   340 345 350
   Leu Pro Pro Gly Ala Asn Ile Val Ile Ile Ala Cys Asp Ser Gly Ser
   355 360 365
   Arg His Leu Ser Lys Phe Trp Lys Glu Ala Lys Gln Ile Glu Tyr Asp
   370 375 380
   Ile Ser Leu Lys Glu Ile Thr Gly Gly Ile Arg
   385 390 395
<210> 3
   <211> 40
   <212> DNA
   <213> Artificial
<220>
   <223> Primer
<400> 3
   gagctcatag cggccatgtc cagttcacga gataagaacg 40
<210> 4
   <211> 42
   <212> DNA
   <213> Artificial
<220>
   <223> Primer
<400> 4
   ggatcctatg cggccgcgga aaaactaaca aggaattcag aa 42
<210> 5
   <211> 1182
   <212> DNA
   <213> Yeast
<400> 5
   atgtcctgtt ctcaaaataa gacttctgtc agtctggcct ggcgtgaatg catatccatc     60
   gcttccgtcc taattggcgc gtatgcatca tacagatact ataagttgtt caaaacacgc    120
   gatattccac gtccaaaaga aggcattgaa gaactcatag gaaacactcc tctggttaag    180
   atcagatctc ttaccaaggc tactggagtt aacatttacg ctaagttgga gttatgcaac    240
   ccagcaggaa gtgctaagga tagagtggct ttaaacatca taaagacggc tgaggaatta    300
   ggcgagctcg tcagaggtga acctggctgg gtatttgaag gaacaagtgg gtcgactggt    360
   atctctatag cagtggtctg taatgcgttg ggttataggg cacacatttc tttgcccgat    420
   gacacatcgc tagaaaaatt ggcattacta gaaagcctcg gtgctactgt taataaggtt    480
   aagcccgcta gtattgtcga tccaaaccaa tacgtaaatg ccgccaaaaa ggcatgtaat    540
   gaactaaaga agagtggtaa tggaataaga gcagtttttg ccgatcagtt cgaaaacgaa    600
   gccaactgga aagtacatta ccagaccaca ggcccagaaa ttgcccatca aactaaggga    660
   aatattgacg cattcattgc tggttgcggt actggtggca ccataactgg tgtggcaaaa    720
   ttcttaaagg aaagagcaaa gataccttgc catgtcgttc tagcggatcc acaaggatct    780
   ggcttttata atagagtcaa ctatggcgtg atgtatgact atgtagagaa ggaaggtact    840
   agaagaaggc atcaagttga cactatcgta gaagggattg ggttaaacag aatcactcat     900
   aattttcata tgggcgaaaa atttattgat gaatcaatac gagtcaacga taatcaagcc    960
   attagaatgg caaaatacct atcggtaaac gacgggctat tcgtggggag tagtacagct   1020
   attaatgcgg tagccgctat tcaggttgca aaaacgcttc ctcacggctc aaacattgtg   1080
   attattgcat gcgacagcgg ttcaagacac ctaagtaaat tctggaaaga agctaaagaa   1140
   atcgatcacg acgtatcatt agaagaagta ataaatatct aa                       1182
<210> 6
   <211> 393
   <212> PRT
   <213> Yeast
<400> 6
   Met Ser Cys Ser Gln Asn Lys Thr Ser Val Ser Leu Ala Trp Arg Glu
   1 5 10 15
   Cys Ile Ser Ile Ala Ser Val Leu Ile Gly Ala Tyr Ala Ser Tyr Arg
   20 25 30
   Tyr Tyr Lys Leu Phe Lys Thr Arg Asp Ile Pro Arg Pro Lys Glu Gly
   35 40 45
   Ile Glu Glu Leu Ile Gly Asn Thr Pro Leu Val Lys Ile Arg Ser Leu
   50 55 60
   Thr Lys Ala Thr Gly Val Asn Ile Tyr Ala Lys Leu Glu Leu Cys Asn
   65 70 75 80
   Pro Ala Gly Ser Ala Lys Asp Arg Val Ala Leu Asn Ile Ile Lys Thr
   85 90 95
   Ala Glu Glu Leu Gly Glu Leu Val Arg Gly Glu Pro Gly Trp Val Phe
   100 105 110
   Glu Gly Thr Ser Gly Ser Thr Gly Ile Ser Ile Ala Val Val Cys Asn
   115 120 125
   Ala Leu Gly Tyr Arg Ala His Ile Ser Leu Pro Asp Asp Thr Ser Leu
   130 135 140
   Glu Lys Leu Ala Leu Leu Glu Ser Leu Gly Ala Thr Val Asn Lys Val
   145 150 155 160
   Lys Pro Ala Ser Ile Val Asp Pro Asn Gln Tyr Val Asn Ala Ala Lys
   165 170 175
   Lys Ala Cys Asn Glu Leu Lys Lys Ser Gly Asn Gly Ile Arg Ala Val
   180 185 190
   Phe Ala Asp Gln Phe Glu Asn Glu Ala Asn Trp Lys Val His Tyr Gln
   195 200 205
   Thr Thr Gly Pro Glu Ile Ala His Gln Thr Lys Gly Asn Ile Asp Ala
   210 215 220
   Phe Ile Ala Gly Cys Gly Thr Gly Gly Thr Ile Thr Gly Val Ala Lys
   225 230 235 240
   Phe Leu Lys Glu Arg Ala Lys Ile Pro Cys His Val Val Leu Ala Asp
   245 250 255
   Pro Gln Gly Ser Gly Phe Tyr Asn Arg Val Asn Tyr Gly Val Met Tyr
   260 265 270
   Asp Tyr Val Glu Lys Glu Gly Thr Arg Arg Arg His Gln Val Asp Thr
   275 280 285
   Ile Val Glu Gly Ile Gly Leu Asn Arg Ile Thr His Asn Phe His Met
   290 295 300
   Gly Glu Lys Phe Ile Asp Glu Ser Ile Arg Val Asn Asp Asn Gln Ala
   305 310 315 320
   Ile Arg Met Ala Lys Tyr Leu Ser Val Asn Asp Gly Leu Phe Val Gly
   325 330 335
   Ser Ser Thr Ala Ile Asn Ala Val Ala Ala Ile Gln Val Ala Lys Thr
   340 345 350
   Leu Pro His Gly Ser Asn Ile Val Ile Ile Ala Cys Asp Ser Gly Ser
   355 360 365
   Arg His Leu Ser Lys Phe Trp Lys Glu Ala Lys Glu Ile Asp His Asp
   370 375 380
   Val Ser Leu Glu Glu Val Ile Asn Ile
   385 390
<210> 7
   <211> 40
   <212> DNA
   <213> Artificial
<220>
   <223> Primer
<400> 7
   gagctcatag cggccatgtc ctgttctcaa aataagactt 40
<210> 8
   <211> 42
   <212> DNA
   <213> Artificial
<220>
   <223> Primer
<400> 8
   ggatcctatg cggccgccca cggaaagtag caaaaaatgt ag 42